# EUROPEAN PATENT APPLICATION

(11) **EP 4 497 827 A1**
(43) Date of publication of application: **29.01.2025**
(21) Application number: 23775178.9
(22) Date of filing: 23.02.2023
(51) Int. Cl.: C12N 15/70, C12N 15/52, C12N 9/02, C12N 9/10, C12N 9/04, C12N 1/20, C12P 7/18

(54) **RECOMBINANT E.COLI STRAIN PRODUCING 1,3-BUTANEDIOL FROM GLUCOSE AND METHOD FOR PRODUCING 1,3-BUTANEDIOL USING SAME**

(30) Priority: 22.03.2022 KR 20220035217; 20.07.2022 KR 20220089507
(71) Applicant: Activon Co., Ltd., Cheongju-si, Chungcheongbuk-do 28104 (KR)
(72) Inventor: PARK, Sunghoon, Ulju-gun Ulsan 44936 (KR); ISLAM, Tayyab, Ulju-gun Ulsan 44919 (KR); NGUYEN, Thuan Phu, Ulju-gun Ulsan 44919 (KR)
(74) Representative: Mewburn Ellis LLP
(86) International application number: PCT/KR2023/002597
(87) International publication number: WO 2023/182679

(57) **Abstract**

The present invention relates to a recombinant E. coli strain producing 1,3-butanediol from glucose and a method for producing 1,3-butanediol using same, in which a recombinant E. coli strain was developed in which a gene of a pathway necessary for biosynthesis of 1,3-BDO from acetyl-CoA is cloned, and a gene of a pathway that interferes with or competes with this pathway is removed. Additionally, an E. coli strain was developed in which the pathways and genes involved in the conversion of glucose to acetyl CoA, the regeneration rate of NADPH used as a cofactor, and the efficient use of the TCA cycle pathway, which are necessary to improve the biosynthetic efficiency of 1,3-BDO, are modified and optimized. Accordingly, the recombinant E. coli strain of the present invention can produce 1,3-butanediol from glucose at a high concentration, yield, and rate.

## Description

### Technical Field

The present disclosure relates to a recombinant E. coli strain producing 1,3-butanediol from glucose and a method of producing 1,3-butanediol using the same.

### Background Art

1,3-Butanediol (1,3-BDO), also known as butylene glycol, is an unnatural alcohol used as a building block to produce industrial chemicals required for cosmetics, food, and pharmaceuticals. In the food industry, it is used as an additive, flavoring agent, solvent or solubilizer for flavoring agent, stabilizer, emulsifier, and antimicrobial agent and preservative. In the pharmaceutical industry, it is also used as a solvent for parenteral drugs while (R)-1,3-BDO is utilized as a precursor for synthesis of azetidinone derivatives, the main chiral intermediates for synthesis of penem and carbapenem β-lactam antibiotics. 1,3-Butanediol has been developed for many uses, especially in the cosmetics industry. It is used as a softener, humectant, anti-crystallizer for insoluble ingredients, solubilizer for ingredients with low water solubility such as scents, antimicrobial agents, and preservatives. For example, it may be used as a moisturizer in hairsprays and setting lotions, reduces the scent loss of essential oils, prevents spoilage by microorganisms, and thus is used as a solvent for benzoate. 1,3-Butanediol may be used in a variety of concentrations, from less than 0.1% to greater than 50%. In terms of cosmetic products, it is used in hair and bath products, eye and facial makeups, perfumes, personal hygiene products, and shaving and skin care preparations.

Due to the increasing demand for 1,3-BDO, several studies on production methods that provide high yields and environmentally friendliness have been conducted. Chemically, 1,3-BDO is produced mainly from petroleum-derived substances such as parochial precursors, acetaldehyde, and 4-hydroxy-2-butanone (4H2B) and has already been commercialized. In this process, some environmentally harmful reagents are used, with involvement of harsh reaction conditions. In contrast, the biocatalytic process offers advantages of being renewable, leaving fewer by-products and no residual metals in the final product. However, bioprocessing has not yet been commercialized due to low productivity and economic feasibility.

1,3-BDO is an unnatural substance, of which naturally occurring metabolic pathway for production thereof has not yet been identified. Therefore, the production of 1,3-BDO through a biotechnological approach relies on the development of recombinant bacterial strains, including genetic engineering and metabolic engineering. It has been reported that the use of strain and path engineering strategies had led to production of 2.4 g/L of R-1,3-BDO with 11% of maximum theoretical yield from glucose in a bioreactor. In addition, Kataoka et al. in Japan reported that they produced up to 9.05 g/l (100.4 mM) for 98 hours. Recently, Liu et al. in China produced 13 g/L of 1,3-BDO under aerobic conditions through a pathway in which 3-hydroxybutyrate is used as an intermediate. GENOMATICA, famous for its biological production of 1,4-BDO, has announced that it has produced 54 g/L of 1,3-BDO, but no technical details are known to support this. Despite the achievement in the research so far, bioprocessing lags far behind chemical processes, and for commercialization of bioprocessing, significant improvements are needed in terms of titer, productivity, and yield.

### Disclosure of the Invention

### Technical Goals

An object of the present disclosure is to provide a recombinant E. coli strain in which a gene of a pathway required for biosynthesizing 1,3-BDO from acetyl-CoA is cloned and from which a gene of a pathway that interferes with or competes with the pathway is removed. In addition, it is to provide a recombinant E. coli strain with modified and optimized pathways and genes involved in conversion of glucose to acetyl-CoA that is required to improve biosynthetic efficiency of 1,3-BDO, regeneration rate of NADPH which is used as a cofactor, and efficient utilization of TCA cycles.

In addition, another object of the present disclosure is to provide a method of enhancing production of 1,3-BDO, including culturing the recombinant E. coli strain. Technical Solutions

In order to achieve the above objectives, the present disclosure provides a recombinant expression vector including a butyraldehyde dehydrogenase (bld) gene, an acetyl-CoA acetyltransferase (phaA) gene, an acetoacetyl-CoA reductase (phaB) gene, and a 3-hydroxybutyraldehyde (3-HBA) reductase gene in order.

In addition, the present disclosure provides a recombinant E. coli strain which is transformed with the recombinant expression vector, with enhanced production of 1,3-BDO.

In addition, the present disclosure provides a method of enhancing production of 1,3-BDO, including culturing the recombinant E. coli strain.

### Advantageous Effects

The present disclosure relates to a recombinant E. coli strain that produces 1,3-butanediol from glucose and a method of producing 1,3-butanediol using the same, wherein the recombinant E. coli strain, in which a gene of a pathway required for biosynthesizing 1,3-BDO from acetyl-CoA is cloned and from which a gene of a pathway that interferes with or competes with this pathway is removed, was developed. Further, an E. coli strain with modified and optimized pathways and genes involved in conversion of glucose to acetyl-CoA that is required to improve biosynthetic efficiency of 1,3-BDO, regeneration rate of NADPH which is used as a cofactor, and efficient utilization of TCA cycles. Accordingly, the recombinant E. coli strain of the present disclosure is capable of producing 1,3-butanediol from glucose at high concentrations, yields, and rates.

### Brief Description of Drawings

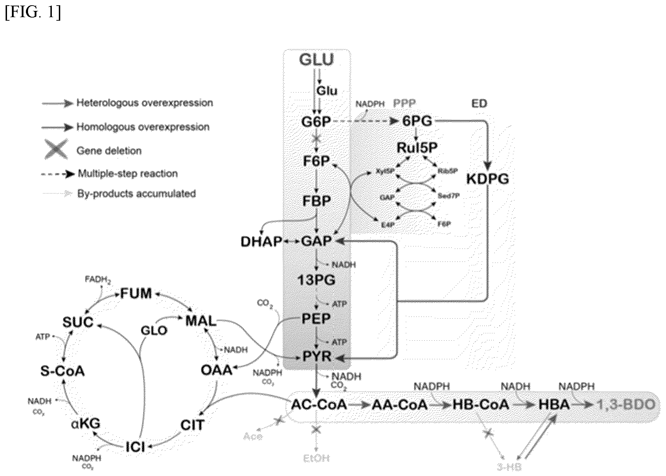

FIG. 1 shows a strategy and main metabolic pathway for construction of a 1,3-butanediol-producing recombinant strain of the present disclosure.
FIG. 2 shows a screening result for 3-hydroxybutyl-CoA (3-HB-CoA) reductase that affects 1,3-BDO production.
FIG. 3 shows a screening result for 3-hydroxybutyraldehyde (3-HBA) reductase.
FIG. 4 shows, through an increase in expression of phaA and phaB genes, that carbon is prone to flow more toward 1,3-BDO production from acetyl-CoA nodes.
FIG. 5 shows performance of strains having a by-product production pathway deleted.
FIG. 6 shows an effect of a volume of fermentation broth on 1,3-butanediol production.
FIG. 7 shows an effect of changes in acetyl-CoA production and TCA cycle on 1,3-BDO production.
FIG. 8 shows effects of deletion of pdhR or gltA genes on 1,3-BDO production through bioreactor culture. A K6-Q10 strain did not undergo deletion in the pdhR gene and gltA gene. OD, optical density; Gluc, glucose; 1,3-BDO, 1,3-butanediol; 3-HB, 3-hydroxybutyric acid; Pyr, pyruvic acid; Suc, succinic acid; EtOH, ethanol; Ace, acetic acid.
FIG. 9 shows toxicity of 3-HBA in inhibiting growth of strains.
FIG. 10 shows differences in performance of three different 3-HBA reductases, namely YqhD, YgjB, and YahK, in the production of 1,3-BDO through bioreactor experiments. YqhD enzyme was used for a K8-Q10 strain, YgjB enzyme for a K8-Q25 strain, and YahK enzyme for a K8-Q10 strain. OD, optical density; Gluc, glucose; 1,3-BDO, 1,3-butanediol; 3-HB, 3-hydroxybutyric acid; Pyr, pyruvic acid; Suc, succinic acid; EtOH, ethanol; Ace, acetic acid.
FIG. 11 shows 1,3-BDO productivity of strains with enhanced ED pathway in glucose metabolism through bioreactor experiments. K8-Q27 strains were those without changes applied to a glycolytic pathway, K22-Q27 strains were △pgi, zwf⁺⁺, △gntR, and K23-Q27 strains were △pgi, zwf⁺⁺, △gntR, edd⁺⁺, and eda⁺⁺. OD, optical density; Gluc, glucose; 1,3-BDO, 1,3-butanediol; 3-HB, 3-hydroxybutyric acid; Pyr, pyruvic acid; Suc, succinic acid; EtOH, ethanol; Ace, acetic acid.
FIG. 12 shows effects of expression of carboxylic acid reductase (CAR) on production of 1,3-butanediol in a bioreactor. The K23-Q27 strains were those without CAR introduced, K23-Q31 strains were those with Mycobacterium marinum-derived Mm_CAR introduced, and K23-Q32 strains were those with Ma_CAR of Mycobacterium abscessus introduced. OD, optical density; Gluc, glucose; 1,3-BDO, 1,3-butanediol; 3-HB, 3-hydroxybutyric acid; Pyr, pyruvic acid; Suc, succinic acid; EtOH, ethanol; Ace, acetic acid.

### Best Mode for Carrying Out the Invention

The present disclosure provides a recombinant expression vector including a butyraldehyde dehydrogenase (bld) gene, an acetyl-CoA acetyltransferase (phaA) gene, an acetoacetyl-CoA reductase (phaB) gene, and a 3-hydroxybutyraldehyde (3-HBA) reductase gene in order.

Preferably, the bld gene may comprise a nucleotide sequence represented by SEQ ID NO: 7, the phaA gene may comprise a nucleotide sequence represented by SEQ ID NO: 5, and the phaB gene may comprise a nucleotide sequence represented by SEQ ID NO: 6, but are not limited thereto.

Preferably, the 3-HBA reductase gene may be, but is not limited to, a yqhD gene comprising a nucleotide sequence represented by SEQ ID NO: 34, a ygjB gene comprising a nucleotide sequence represented by SEQ ID NO: 35, or a yahK gene comprising a nucleotide sequence represented by SEQ ID NO: 36.

More preferably, a ribosome binding site (RBS) in a 5' untranslated region (UTR) of the ygjB gene may comprise a nucleotide sequence represented by SEQ ID NO: 37 or SEQ ID NO: 38, but is not limited thereto.

Preferably, the recombinant expression vector may further include carboxylic acid reductase (CAR) gene and, more preferably, the CAR gene may comprise a nucleotide sequence represented by SEQ ID NO: 20 or SEQ ID NO: 21, but is not limited thereto.

As used herein, the term "vector" refers to a self-replicating DNA molecule used to carry a clonal gene (or other pieces of clonal DNA).

As used herein, the term "expression vector" refers to a recombinant DNA molecule including a desired coding sequence and appropriate nucleic acid sequences essential to express the operably linked coding sequence in a particular host organism. The expression vector may preferably include one or more selection markers. The marker is a nucleic acid sequence with properties that may conventionally be selected by chemical means, including any gene that may distinguish a transformed cell from untransformed cells. Examples include, but are not limited to, genes resistant to antibiotics such as ampicillin, kanamycin, geneticin G418), bleomycin, hygromycin, and chloramphenicol, and it may be appropriately selected by those skilled in the art.

In addition, the present disclosure provides a recombinant E. coli strain with enhanced production of 1,3-Butanediol (1,3-BDO), which is transformed with the recombinant expression vector. The base E. coli strain used in the present disclosure is K12 MG1655, which is the representative strain of the K-group among several E. coli and a generally regarded as safe (GRAS) strain that has been recognized for its safety.

Preferably, the recombinant E. coli strain may be, but is not limited to, one obtained by transforming, with the recombinant expression vector, an E. coli strain from which an alcohol dehydrogenase (adhE) gene, a pyruvate oxidase (poxB) gene, a D-lactate dehydrogenase (ldhA) gene, a phosphate acetyltransferase-acetate kinase (pta-ackA) gene, and an acyl-CoA thioesterase (yciA) gene are deleted.

More preferably, the recombinant E. coli strain may be, but is not limited to, one obtained by transforming, with the recombinant expression vector, an E. coli strain from which a DNA-binding transcriptional dual regulator (pdhR) gene is additionally deleted.

More preferably, the recombinant E. coli strain may be one obtained by transforming, with the recombinant expression vector, an E. coli strain from which a citrate synthase (gltA) gene is additionally deleted, the recombinant E. coli strain may be one obtained by transforming, with the recombinant expression vector, an E. coli strain from which a glucose-6-phosphate isomerase (pgi) gene and a DNA-binding transcriptional repressor (gntR) gene are additionally deleted and in which a NADP+-dependent glucose-6-phosphate dehydrogenase (zwf) gene is additionally overexpressed, and the recombinant E. coli strain may be one obtained by transforming, with the recombinant expression vector, an E. coli strain in which a phosphogluconate dehydratase (edd) gene and a 2-keto-3-deoxygluconate 6-phosphate aldolase (eda) gene are additionally overexpressed, but is not limited thereto.

More preferably, the recombinant E. coli strain may be a strain deposited under the accession number KCTC 15315BP, and the strain deposited under the accession number KCTC 15315BP is the strain listed in this specification as a K23-Q32 strain.

The recombinant E. coli strain prepared in the present disclosure is a recombinant microorganism prepared by the present inventors by introducing the 1,3-BDO biosynthesis pathway and eliminating the competing pathway or the degradation pathway of intermediate metabolites on the 1,3-BDO biosynthesis pathway, wherein the strain uses glucose as a raw material. Furthermore, it is an E. coli strain with modified and optimized pathways and genes involved in conversion of glucose to acetyl-CoA that is required to enhance biosynthetic efficiency of 1,3-BDO, regeneration rate of NADPH used as a cofactor, and efficient utilization of TCA cycles. In this process, new genes are introduced, gene expression levels are optimized, and carbon and energy metabolism is regulated and optimized to improve 1,3-BDO production rate and yield. E. coli K12 does not have an ability to produce 1,3-BDO from glucose, but the recombinant E. coli prepared in the present disclosure successfully produces 1,3-BDO during growth in the presence of glucose.

The genetically recombinant E. coli strain developed by the present inventors is capable of producing 1,3-BDO with superior performance, ensuring a maximum concentration of 80 g/L (883 mM), a glucose yield of 0.74 mol/mol, and a production rate of 1.67 g/L/h.

In addition, the present disclosure provides a method of enhancing production of 1,3-BDO, including culturing the recombinant E. coli strain.

In the present disclosure, a total of 36 genes may be modified, deleted, or upregulated, and the genes modified, used, or deleted in the present disclosure, the names of the proteins encoded thereby, and the sequence numbers are shown in Table 1.

**TABLE 1**

| Sequence Number | Gene | Proteins/Enzymes |
|---|---|---|
| 1 | *ackA* | acetate kinase |
| 2 | *adhE* | fused acetaldehyde-CoA dehydrogenase and iron-dependent alcohol dehydrogenase |
| 3 | *atoB* | acetyl-CoA acetyltransferase |
| 4 | *sfp* | 4'-phosphopantetheinyl transferase |
| 5 | *Cn_phaA* | Acetyl-CoA acetyltransferase*(Cupriavidus necator)* |
| 6 | *Cn_phaB* | Acetoacetyl-CoA reductase*(Cupriavidus necator)* |
| 7 | *Cs_bld* | butyraldehyde dehydrogenase*(Clostridium saccharoperbutylacetonicum)* |
| 8 | *eda* | 2-keto-3-deoxygluconate 6-phosphate aldolase |
| 9 | *edd* | phosphogluconate dehydratase |
| 10 | *frdA* | fumarate reductase flavoprotein subunit |
| 11 | *frdB* | fumarate reductase iron-sulfur protein |
| 12 | *frdC* | fumarate reductase membrane protein FrdC |
| 13 | *frdD* | fumarate reductase membrane protein FrdD |
| 14 | *gltA* | citrate synthase |
| 15 | *gnd* | 6-phosphogluconate dehydrogenase, decarboxylating |
| 16 | *gntR* | DNA-binding transcriptional repressor GntR |
| 17 | *iclR* | DNA-binding transcriptional repressor IclR |
| 18 | *ldhA* | D-lactate dehydrogenase |
| 19 | *lpdA* | lipoamide dehydrogenase |
| 20 | *Ma_CAR* | Carboxylic acid reductase*(Mycobacterium abscessus)* |
| 21 | *Mm_CAR* | Carboxylic acid reductase*(Mycobacterium marinum)* |
| 22 | *maeB* | malate dehydrogenase (oxaloacetate-decarboxylating) (NADP+) |
| 23 | *pdhR* | DNA-binding transcriptional dual regulator PdhR |
| 24 | *pgi* | glucose-6-phosphate isomerase |
| 25 | *pntA* | pyridine nucleotide transhydrogenase subunit α |
| 26 | *pntB* | pyridine nucleotide transhydrogenase subunit β |
| 27 | *poxB* | pyruvate oxidase |
| 28 | *ppc* | phosphoenolpyruvate carboxylase |
| 29 | *pta* | phosphate acetyltransferase |
| 30 | *tesB* | acyl-CoA thioesterase II |
| 31 | *udhA* | soluble pyridine nucleotide transhydrogenase |
| 32 | *yciA* | acyl-CoA thioesterase YciA |
| 33 | *zwf* | NADP+-dependent glucose-6-phosphate dehydrogenase |
| 34 | *yqhD* | 3-hydroxybutyraldehyde reductase*(E. coli)* |
| 35 | *ygjB* | 3-hydroxybutyraldehyde reductase*(E. coli)* |
| 36 | *yahK* | 3-hydroxybutyraldehyde reductase*(E. coli)* |

Hereinafter, the present disclosure will be described in more detail through examples. It will be apparent to those skilled in the art that these examples are merely intended to describe the present disclosure more specifically, and the scope of the present disclosure is not limited to the following examples according to the gist of the present disclosure.

### Modes for Carrying Out the Invention

Hereinafter, the present disclosure will be described in more detail through examples. However, the present disclosure is not limited by these examples.

### <Experimental Example>

The following experimental examples are intended to provide experimental examples that are commonly applied to each example according to the present disclosure.

### 1. Materials and methods

All restriction enzymes, highly reliable DNA polymerase, and other DNA modifying enzymes for gene cloning were purchased from New England Bio-Labs (Beverly, MA, USA). Mini plasmid isolation kit, DNA gel extraction kit, and genomic DNA isolation kit were purchased from Cosmotech Co. Ltd. (Seoul) and Promega (Madison, Wisconsin, USA), respectively. RNA isolation kit was purchased from Qiagen (Mannheim, Germany). The iScript cDNA Synthesis Kit and SyBr green RT-PCR master mix were purchased from BioRad (Seoul, Korea). Oligonucleotide synthesis and DNA sequencing were performed by Macrogen Co., Ltd. (Seoul, South Korea). Tryptone and yeast extracts were purchased from Difco (Becton Dickinson, Franklin Lakes, NJ, USA). Glycerol, glucose, and all other chemicals and enzymes were purchased from Sigma-Aldrich (St. Louis, MO, USA) unless otherwise specified herein.

### 2. Construction of gene recombinant strains and plasmids

Including existing strains and plasmids, the recombinant strains and plasmids in Table 2 below were prepared and used. Here, genetic recombination was carried out as follows. For overexpression and development of deletion-induced plasmids, E. coli DH5α was used. Chromosomal genetic manipulation was performed using the in-frame tagged deletion/insertion method. In brief, 500 bp in the upstream (upstream fragment, A) and downstream (downstream fragment, B) regions of a target gene were amplified by PCR and linked using the overlapping PCR method. Subsequently, fragment AB was cloned using NotI and XbaI or pKOV vectors at BamHI restriction sites. The resulting plasmids were used to delete the target gene from the chromosomal DNA by homology recombination. Finally, the deletion-induced mutant strains were screened via PCR and observed by sequencing. For the replacement of all target genes, the target gene was first removed, and then the gene of interest was inserted by a similar in-frame tagging method.

The other recombinant strains shown in Table 2 were prepared in the same way, and the restriction enzymes and primers used to make the deletion-induced plasmids (pKOVs) used therefor are as shown in Tables 3 and 4.

**TABLE 2**

| Host cells | | Plasmids | |
|---|---|---|---|
| Strain name | Genotypes | Name | Phenotypic characteristics |
| K | *E. coli K12 MG1655* | pC | *pCDF- duet* |
| W3110 | *E. coli W3110* | pQ | *pQE80L -empty* |
| BL21 | *E. coli BL21* | pC1 | *pCDF-phaA-phaB* |
| BW25113 | *E. coli BW25113* | pQ1 | *pQE80L -Cs_bld* |
| W | *E. coli W* | pQ2 | *pQE80L -Se_pduP* |
| XL1-Blue | *E. coli XLI-Blue* | pQ3 | *pQE80L -Kp_pduP* |
| K2 | *ΔldhA ΔpoxB ΔadhE* | pQ4 | *pQE80L -Lr_pduP* |
| K3 | *K2 & Δpta-ackA* | pQ5 | *pQE80L -Ca_mcrC* |
| K4 | *K3 & ΔatoB* | pQ6 | *pQE80L -Cs_bld-bdh* |
| K5 | *K3 & ΔtesB* | pQ7 | *pQE80L -Cs bld-yqhD* |
| K6 | *K3 & ΔyciA* | pQ8 | *pQE80L -Cs bld-dhat* |
| K7 | *K3 & ΔtesB, ΔyciA* | pQ9 | *pQE80L -Cs_bld-pduQ* |
| K8 | *K5 & ΔpdhR* | pQ10 | *pQE80L -Cs_bld-phaA-phaB-yqhD* |
| K9 | *K5 & Ec_lpdA :: Kp_lpdA(E354K)* | pQ11 | *pQE80L -Ca_mcrC* |
| K10 | *K5 & Ec_lpdA :: Kp _lpdA(E354K) & ΔpdhR* | pQ12 | *pQE80L -Cs_bld* |
| K11 | *K10 & ΔfrdABCD* | pQ13 | *pQE80L -Cs_pduQ* |
| K12 | *K10 & ΔgltA* | pQ14 | *pQE80L -Ca_bdh* |
| K13 | *K10 & Δppc* | pQ15 | *pQE80L -Ec_yqhD* |
| K14 | *K10 & ΔiclR* | pQ16 | *pQE80L -Kp_dhaT* |
| K15 | *K8 & ΔpntAB* | pQ17 | *pQE80L -Ca_mcrN* |
| K16 | *K8 & ptrc_pntAB* | pQ18 | *pQE80L -AKR (P.d 13867)* |
| K17 | *K8 & ΔudhA* | pQ19 | *pQE80L -AKR (P.d 19244)* |
| K18 | *K8 & Δpgi* | pQ20 | *pQE80L -AKR (P.p)* |
| K19 | *K18 & ptrc_zwf* | pQ21 | *pQE80L -AKR (P.a)* |
| K20 | *K19 & ptrc_gnd* | pQ22 | *pQE80L -yjgB* |
| K21 | *K20 & Δedd-eda* | pQ23 | *pQE80L -yahK* |
| K22 | *K19 & ΔgntR,* | pQ24 | *pQE80L -Cs bld-phaA-phaB-AKR (P.a)* |
| K23 | *K21 & ptrc*_*edd-eda* | pQ25 | *pQE80L -Cs bld-phaA-phaB-yjgB* |
| | | pQ26 | *pQE80L -Cs bld-phaA-phaB-yahK* |
| | | pQ27 | *pQE80L -Cs bld-phaA-phaB-RBS1-yjgB* |
| | | pQ28 | *pQE80L -Cs bld-phaA-phaB-RBS2-yjgB* |
| | | pQ29 | *pQE80L -Cs bld-phaA-phaB-RBS3-yjgB* |
| | | pQ30 | *pQE80L -Cs bld-phaA-phaB-RBS4-yjgB* |
| | | pQ31 | *pQE80L -Cs_bld-phaA-phaB-RBS1-yjgB-MmCAR_sfp* |
| | | pQ32 | *pQE80L -Cs bld-phaA-phaB-RBS1-yjgB-MaCAR_sfp* |

In Table 2 above, "△" represents the deletion of the gene. In addition, in Table 2, "::" represents nucleotide sequence manipulation of the gene. For example, "lpdA::lpdAE324K" means that, regarding a gene encoding a gene of LpdA protein, the lpdA gene, which encodes the protein in which the 324^{th} amino acid residue of the LpdA protein is modified from E to K, is engineered, and the existing lpdA gene was substituted with the engineered lpdA gene.

**TABLE 3**

| Gene | Restriction enzymes |
|---|---|
| *ackA* | *NotI and XbaI* |
| *adhE* | *NotI and XbaI* |
| *atoB* | *NotI and XbaI* |
| *bld* | *BamhI and KpnI* |
| *CAR* | *Sbfl and HindiIII* |
| *eda* | *NotI and XbaI* |
| *edd* | *NotI and XbaI* |
| *frdA* | *NotI and XbaI* |
| *frdB* | *NotI and XbaI* |
| *frdC* | *NotI and XbaI* |
| *frdD* | *NotI and XbaI* |
| *gltA* | *NotI and XbaI* |
| *gnd* | *NotI and XbaI* |
| *gntR,* | *NotI and XbaI* |
| *iclR* | *NotI and XbaI* |
| *ldhA* | *NotI and XbaI* |
| *lpdA::lpdAE354 K* | *NotI and XbaI* |
| *maeB* | *NotI and XbaI* |
| *pdhR* | *NotI and XbaI* |
| *pgi* | *NotI and XbaI* |
| *phaA* | *KpnI and SacI* |
| *phaB* | *SacI and NotI* |
| *pntA* | *NotI and XbaI* |
| *pntB* | *NotI and XbaI* |
| *poxB* | *NotI and XbaI* |
| *ppc* | *NotI and XbaI* |
| *pta* | *NotI and XbaI* |
| *tesB* | *NotI and XbaI* |
| *udhA* | *NotI and XbaI* |
| *yciA* | *NotI and XbaI* |
| *yqhD* | *NotI and HindIII* |
| *zwf* | *NotI and XbaI* |

**TABLE 4**

| Gene | Primer sequence |
|---|---|
| *ldhA* | FP: CTACGGATCCTCGCTTCCGCCAGCCTCGGACATTTCCTG |
| | RP: AACAGCGGCCGCGTTTTCCGTCAGATCGACCTGCGCACCCTC |
| *poxB* | FP: ATCTAAGCGGCCGCCACCTTACCGTTATGGGTGTTTTC |
| | RP: CTGAAGGATCCAGTCTATTTCTTTGTTGGCGCACTGG |
| *adhE* | FP: AAATCTAGAGGCTTCGGCCTCGCGGAGAATACA |
| | RP: TATGCGGCCGCTGGATGAAAGCCAGTTTCTGA |
| *Pta-ackA* | FP: GATCGCGGCCGCCTGCATCGGCAGGCTCAGCGCAAACAG |
| | RP: CTAGAGGATCCGCGGACTGAATAGCGATTTCCGCCAG |
| *atoB* | FP: CGGGGATCGCGGCCGCTACATAAAACGCCAATGGCTTATATG |
| | RP:GGTCGACTCTAGACATCAGCCCGGATGGGCAAAGCCG |
| *tesB* | FP: GGGGATCGCGGCCGCTACCTTCAGTACGCACCGCTTTCTG |
| | RP: CGACTCTAGACACTGCTGGGGGCGTGTTCTGG |
| *yciA* | FP: CCCGGGGATCGCGGCCGCAAGATGGCCCTGATCACTTTTGTTC |
| | RP: GTCGACTCTAGATGGTCATTGAAAAGCCGAAGCCGAAACC |
| *pdhR* | FP: |
| | GGTACCCGGGGATCGCGGCCGCATGCGTGTGTAAGTTTGCAAT TCCG |
| | RP: |
| | CATTCTCCGGTCGACTCTAGAAAGACTGGAAGGACGCCATATG GC |
| *lpdA::lpdAE3 54 K* | FP: GGTACCCGGGGATCGCGGCCGCTCGGCGTTTCCAAGTCCGCG |
| | RP: CATTCTCCGGTCGACTCTAGACCAGGCAGTTCGCGAGAGAATG |
| *frdABCD* | FP: |
| | CGGTACCCGGGGATCGCGGCCGCCGTGTCTCAAACGGGACCA AATG |
| | RP: |
| | CGCCATTCTCCGGTCGACTCTAGAGTCGCTTGAGGATTTCACCT C |
| *gltA* | FP: CGGTACCCGGGGATCGCGGCCGCTGTTCCGGAGACCTGGCG |
| | RP: |
| | CATTCTCCGGTCGACTCTAGAAACCTCCCCCTTAAAGTGAATTT C |
| *ppc* | FP: |
| | CGGTACCCGGGGATCGCGGCCGCACCTACAGTGACTCAAACG ATGC |
| | RP: |
| | CATTCTCCGGTCGACTCTAGATTTCGAACATCACAAACAGAAC G |
| *iclR* | FP: |
| | CGGTACCCGGGGATCGCGGCCGCCCGACAGGGATTCCATCTGG TAATC |
| | RP: CATTCTCCGGTCGACTCTAGATATGACGACCATTTTGTCTACAG |
| *pntAB* | FP: |
| | GTACCCGGGGATCGCGGCCGCAAACCAGGTACTGGTATTGTTA TTAAC |
| | RP: |
| | CATTCTCCGGTCGACTCTAGAAACGTGCGATTGACAGTGTTTCT C |
| *udhA* | FP: GTACCCGGGGATCGCGGCCGCTCTACGTCGCGGAAATGCCG |
| | RP: CATTCTCCGGTCGACTCTAGACGCAACTGGACAGCGGCAAAC |
| *pgi* | FP: |
| | CGGTACCCGGGGATCGCGGCCGCTTTTCAGCCTTGGCACAAGG GAAG |
| | RP: |
| | CCATTCTCCGGTCGACTCTAGAATAACAATTTCCCTTCATTGAA TGAATGG |
| *maeB* | FP: |
| | GTACCCGGGGATCGCGGCCGCCAGCACCGAAATTGACCGCCA G |
| | RP: CATTCTCCGGTCGACTCTAGAATAATTGCCGTGCCGTGCTGATC |
| *zwf* | FP: GTACCCGGGGATCGCGGCCGCTCTAGGGCGGCGGATTTGTCC |
| | RP: |
| | CCATTCNCCGGTCGACTCTAGACTGGATTCTCACCAATAAAAA ACG |
| *gnd* | FP: |
| | CCCGGGGATCGCGGCCGCCCCGCTTATTCGCACCTTCCTTAAT AAAAC |
| | RP: |
| | CATTCTCCGGTCGACTCTAGAGTCGGTTGGAGTGGCGATGATG ACATAATC |
| *edd-eda* | FP: |
| | CCCGGGGATCGCGGCCGCACCACAAACATAACCTGCAAATCA C |
| | RP: |
| | GAAATTGAGTTGTCGCGGCGCTGTTTCCTGTGTGAAATTGTTAT CC |
| *gntR* | FP: GTACCCGGGGATCGCGGCCGCCAAACAGCTGGTGCTCTCGCC |
| | RP: |
| | CATTCTCCGGTCGACTCTAGAAGGCGGCTTTCAATCACATCAA AATC |
| *Ma_CAR* | FP: AAATCTAGAGGCTTCGGCCTCGCGGAGAATACA |
| | RP: GGGGATCGCGGCCGCTACCTTCAGTACGCACCGCTTTCTG |
| *Mm_CAR* | FP: CATTCTCCGGTCGACTCTAGATATGACGACCATTTTGTCTACAG |
| | RP: TATGCGGGGACGCCGCTGGATGAAAGCCAGTTTCTGA |

### 3. Culture condition

For culture in a shake flask, cells were cultured in agar LB medium for about 10 to 11 hours to prepare a primary inoculum. In the primary inoculation, the cells were transferred to a freshly prepared modified M9 medium and cultured for about 10 hours to prepare a secondary inoculum. The main culture for the production of 1,3-BDO was initiated by inoculating the secondary inoculum into the same modified M9 medium. The components of the modified M9 medium used in the present disclosure are 1.0 g/L of an yeast extract added with 100 mM potassium phosphate buffer (pH 7.0), 1.0 g/L of NH₄SO₄, and 0.25 g/L of MgSO₄ . 7H₂O. 50 g/L of glucose was used as a carbon source. For recombinant cells, kanamycin (50 µg/ml) was added for stability of plasmids whenever needed. For gltA-deficient strains, 20 g/L of sodium glutamate was added. All strains were cultured in a 250 mL Erlenmeyer flask with a medium volume of 20 mL and cultured at 37°C/100 rpm in an incubator shaker. In all experiments, the starting OD600 for strain culture was 0.1, and 0.25 mM IPTG was added for expression of the 1,3-BDO pathway. In the case of two-stage culture, the culture was stopped at the end of the exponential growth, and cells were harvested via centrifugation.

In the case of the two-stage culture, in other words, when an experiment, in which the strain culture in the first stage and the production of 1,3-BDO in the second stage are separated, is progressed, LB or modified M9 medium was usually used in the first stage to facilitate strain growth; and in the second stage, the strains cultured in the first stage were harvested to remove the residual culture medium components through centrifugation and washing and to facilitate the production of 1,3-BDO in the presence of phosphate buffer solution (pH 7.0) and glucose in a state where the nitrogen source was very low or deficient. In the case of the second stage, since there was almost no bacterial growth, the stage was proceeded to a state similar to bioconversion.

Bioreactor experiments were performed in the Effendroff bioreactor (Germany, 1.0 L modified M9 medium, 2.5 L vessel). The starters were prepared in the same way as described for the flask experiment. The air injection rate was set to 1.0 vvm, and the stirring speed was adjusted in the range of 200-700 rpm to maintain dissolved oxygen (DO) at 10%. Glucose was maintained at 50 to 250 mM by intermittent addition. By adjusting the concentration of nitrogen or carbon sources in the medium without strain acquisition or washing, cell growth and 1,3-BDO production steps were properly divided.

### 4. Measurement of enzymatic activities

The activity of 3-HB-CoA reductase was measured as described in the document. The reaction mixture was prepared to include 50 mM Tris-HCl, pH 7.0, 0.15 mM NADH, and 0.3 mM 3-hydroxybutyryl-CoA as substrates. Purified enzymes were added, and the reduction in the NADH concentration at 37°C was continuously monitored by a photometer at 340 nm.

The activity of alcohol dehydrogenase was determined by photometric monitoring of the increase/decrease in NADP+/NADPH at 340 nm. The reaction mixture included 50 mM Tris-HCl at pH 7.0. For measurement of forward reactions, preparation was followed to include 0.15 mM NADPH and 5 mM 3-hydroxybutyraldehyde as substrates at 37°C. For reverse reactions, 2 mM NADP+ and 30 mM 1, 3-BDO were used as cofactors and substrates, respectively. For the reduction reaction, 1 unit of enzymatic activity was defined as the amount of enzyme required to oxidize 1 µmol of NAD(P)H to NADP+ for 1 minute. For oxidation reactions, 1 unit of enzymatic activity was defined as the amount of enzyme required to reduce 1 µmol of NAD(P)+ to NAD(P)H for 1 minute.

### 5. Analytical method

Cell concentrations were measured in absorbance (OD 600) at 600 nm in a 10 mm path-length cuvette using a double-beam spectrophotometer (Lambda 20, PerkinElmer, Norwalk, CT, USA). Protein concentrations were measured on a microtiter plate reader (Synergy H1, Bioteck) using the Bradford method using bovine serum albumin (BSA) as a standard. Concentrations of glycerol, glucose, 1,3-PDO, and other metabolites were measured using high-performance liquid chromatography (HPLC, Agilent 1260 infinity II, USA). For liquid chromatographic analysis, the supernatant obtained by centrifugation of the culture sample at 10000 × g for 10 minutes was filtered with Tuffryn membrane (Acrodisc, Pall Life Sciences) and isolated through 300-mmx7.8-mm Aminex HPX-87H column (Bio- Rad, USA) using 2.5 mmol/L H2SO4 as a mobile phase at 65°C.

### <Example 1> Construction of a base strain and screening of 3-hydroxybutyl-CoA (3-HB-CoA) reductase

When grown in glucose, E. coli produces acetyl-CoA. Therefore, the following four enzymes are needed to produce acetyl-CoA, or 1,3-BDO in the presence of glucose. (1) β-Ketothiolase (also called acetyl-CoA acetyltransferase), which condenses/converts two acetyl-CoAs into one acetoacetyl-CoA, (2) acetoacetyl-CoA reductase which converts acetoacetyl-CoA to β-hydroxybutyryl-CoA through reduction, (3) β-hydroxybutyryl-CoA reductase which converts β-hydroxybutyryl-CoA to 3-hydroxybutyraldehyde (3-HBA), and (4) 3-HBA reductase which converts 3-HBA to 1,3-BDO through reduction. Of the four reactions, the reactions (2) ~ (4) require NADPH or NADH as the reducing power. Selected as base strains were a PhaA enzyme encoded by the Cn_phaA of Cupriavidus necator as the first enzyme, a PhaB enzyme encoded by the Cn_phaB of Cupriavidus necator as the second enzyme, and the Bld enzyme encoded by the Cs_bld of Clostridium saccharoperbutylacetonicum as the third enzyme. The fourth 3-HBA reductase was not selected, but the aldehyde dehydrogenase possessed by the host cell, E. coli, was allowed to catalyze this reaction.

Two recombinant plasmids, pC1 and pQ1, were developed and introduced into E. coli K12. Construction was performed by making pC1 derived from pCDF plasmids with a medium-copy number express the Cn_phaB and Cn_phaA of Cupriavidus necator under the control of the Ptet promoter, while having pQ1 derived from pQE80L plasmids with a high-copy number express Cs_bld of Clostridium saccharoperbutylacetonicum under the control of T5 promoter. These two plasmids were then introduced into the wild-type E. coli K12, and the strain was named K1-C1Q1.

The 1,3-BDO productivity of K1-C1Q1 was investigated through a two-stage culture. First, the strains were cultured for about 6 hours in LB medium, and then bioconversion was performed in a phosphate buffer solution including 30 g/L of glucose. K1-C1Q1 produced 7.6 mM BDO at 24-hour bioconversion. As a by-product, 3 mM EtOH and 15 mM acetate were produced. On the other hand, the K1-CQ control strain, in which only the empty plasmid was introduced without the 1,3-BDO pathway, did not produce 1,3-BDO at all. These results show that the 1,3-BDO production pathway is essential for 1,3-BDO production, and that the 1,3-BDO biosynthesis pathway developed in the base strain K1-C1Q1 functions properly.

The effects of 3-HB-CoA reductase in the base strain were examined. This enzyme converts 3-HB-CoA to 3-HBA using NAD(P)H as a reducing power, with no reports on the natural enzyme specific to this reaction. Bld was used in the preparation of the base strain K1-C1Q1, but this enzyme is known to be sensitive to oxygen.

Four types of enzymes were investigated, including three types of PduPs known to be resistant to oxygen and the malonyl-CoA reductase MCR-C (derived from C. aurantiacus) (Table 2). These enzymes were cloned in pQE80L plasmids instead of bld. The selected three types of PduPs were Se-pduP (K1-C1Q2) derived from Salmonella enterica, Kp_pduP derived from Klebsiella pneumonia (K1-C1Q3), and Lr_pduP from Lactobacillus reuteri (K1-C1Q4). The Ca_mcrC strain was named K1-C1Q5.

Of the 3-HB-CoA reductase tested, only Se_PduP (derived from S. enterica) provided 1,3-BDO production (6.2 mM) comparable to Bld. However, Se_PduP produced ~3 times more ethanol than Cs_Bld, indicating that Se_PduP has undesirable side reaction activity for acetyl-CoA.

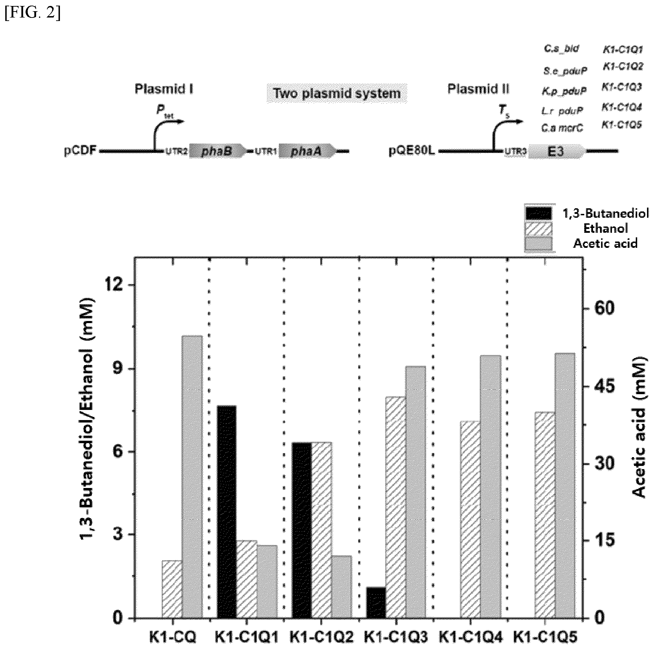

K1-C1Q1 (Cs_Bld) showed high BDO titers and low ethanol production, such that it was chosen for the next stage of strain development (FIG. 2).

### <Example 2> Screening of 3-hydroxybutyraldehyde (3-HBA) reductase

So far, the enzyme for the conversion of 3-HBA to BDO, i.e., the 3-HBA reductase to carry out the fourth step has not been overexpressed, but BDO has been successfully produced. This suggests that the intrinsic aldehyde dehydrogenase or alcohol dehydrogenase (ADH) present in the E. coli K12 host has activity for 3-HBA.

For example, E. coli's ADH, such as Ec_YqhD, has been reported to reduce several aldehydes to the alcohol in order to detoxify aldehyde in a non-specific way.

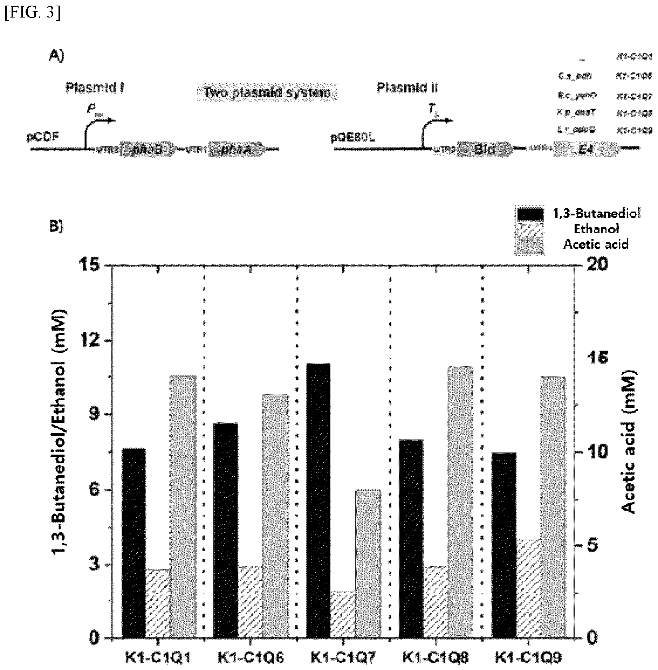

To investigate the effect of overexpression of the 3-HBA reductase on 1,3-BDO production, Ec_YqhD was cloned (K1-C1Q7) along with the bld gene on the pQE80L plasmid to investigate 1,3-BDO production. In addition, investigation was also conducted on the overexpression of three types of genes, including Kp_DhaT derived from Klebsiella pneumoniae (K1-C1Q8), Lr_PduQ from Lactobacillus reuteri (K1-C1Q9), and Cs_Bdh from Clostridium saccharoperbutylacetonicum (K1C1Q6). Thereamong, Kp_DhaT and Lr_PduQ have been widely used to produce 1,3-propanediol from 3-hydroxypropionaldehyde, and Cs_bld have been used to produce butanol from butyraldehyde. The genes encoding the four types of enzymes, Cs_Bdh(pQ6), Ec_YqhD(pQ7), Kp_DhaT(pQ8), and Lr_PduQ(pQ9), were each cloned in the Cs_bld downstream of the pQ1 plasmid and tested for 1,3-BDO production for their recombinant E. coli (FIG. 3).

YqhD (K1-C1Q7) produced the highest amount of 1,3-BDO, with the lowest production of acetate at the same time. A K1-C1Q7 strain that overexpresses YqhD was used for future strain development.

### <Example 3> Expansion of 1,3-BDO production through overexpression of phaA and phaB

In all strains developed so far, acetate (up to ~15 mM) always accumulated as a major by-product. It was hypothesized as a cause that the 'pulling' from the acetyl-CoA node to the 1,3-BDO synthesis pathway was not high enough compared to the pulling toward the TCA cycle, which may be assumed to be due to the low expression of phaA and phaB. The phaA and phaB genes were cloned to pCDF, a medium copy plasmid.

Both the Cn_phaA and Cn_phaB genes were transferred to pQ1 plasmids derived from pQE80L, which are high copy plasmids, so that four genes (Cn_phaA, Cn_phaB, Cs_bld, and Ec_yqhD) were expressed in a single plasmid that is designated as pQ10. Here, the Cs_bld gene was placed at the very front in the pQ1 plasmid because the Cs_Bld enzyme is known to have a low kcat value for 3-HB-CoA which is a substrate.

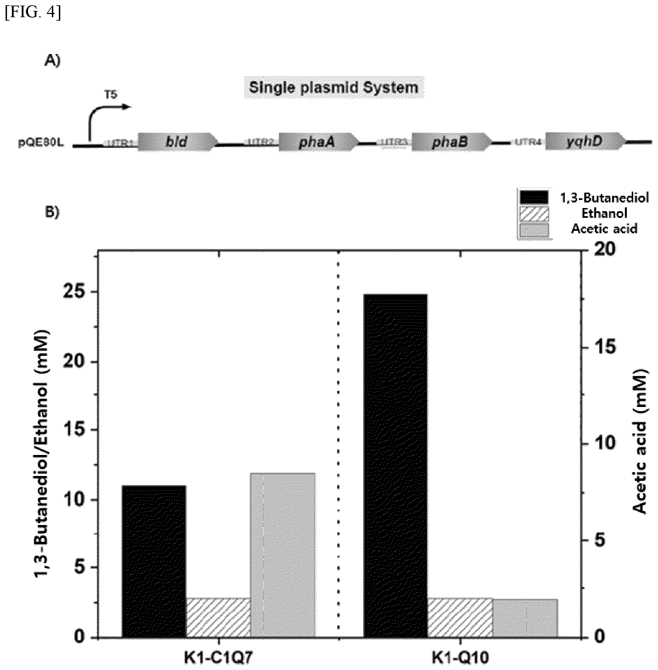

In the K1-Q10 gene recombinant strains in which Cn_phaA and Cn_phaB were transferred to the high copy plasmid, acetate production was significantly reduced from 8 mM to 4 mM (FIG. 4). In addition, the production of BDO increased ~2.5-fold, from 10.7 mM to 25 mM.

### <Example 4> Elimination of by-product production pathways

The following seven genes adhE, poxB, ldhA, pta-ackA, atoB, tesB, and yciA were sequentially removed. First, deletion of lactate dehydrogenase (ldhA), alcohol dehydrogenase (adhE), and pyruviate oxidase (poxB) was performed for the purpose of preventing loss of precursors (PYR, AC-CoA) and cofactors (NADH). Lactate production was eliminated from K2-Q10 strains (ΔadhE, ΔpoxB, ΔldhA), but it did not affect ethanol or acetate production. Ethanol may be produced by the non-specific activity of Bld and/or YqhD for acetyl-CoA and/or acetaldehyde. Acetate may also be produced through acetate phosphatase and kinase (pta-ackA) and/or thioesterase (yciA).

Acetate phosphatase and kinase (pta-ackA) were deleted from the K3-Q10 strain, but acetate production was not reduced. However, the production of 1,3-BDO was enhanced by the deletion of pta-ackA.

Acetyl-CoA acetyltransferase (AtoB) in E. coli is known to have a strong reverse cleavage activity for acetoacetyl-CoA, producing two acetyl-CoAs from acetoacetyl-CoA. If this activity is very high, it may form a so-called 'futile cycle' in which acetyl-CoA and acetoacetyl-CoA continue to circulate together with acetyl-CoA acetyltransferase, which is coded by phaA that catalyzes the forward reaction. However, no positive effects were observed by atoB deletion (K4).

tesB and yciA, which code for thioesterase, remove CoA from the CoA compound and may therefore increase 3-HB or acetate production. In previous studies, these genes were overexpressed for the production of 3-HB. tesB deletions did not reduce 3-HB or acetate production. On the other hand, yciA deletion (K6-Q10) reduced both acetate and 3-HB production, enhanced BDO titers up to ~42 mM, and improved the yield to ~0.42 mol/mol.

However, dual deletion (K7Q10) of tesB & yciA did not show better results than single deletion (K6-Q10) of yciA in reduction of 3-HB or acetate. It is known that there are many thioesterases that induce 3-HB production. However, in this study, only yci deletion was effective in reducing 3-HB production.

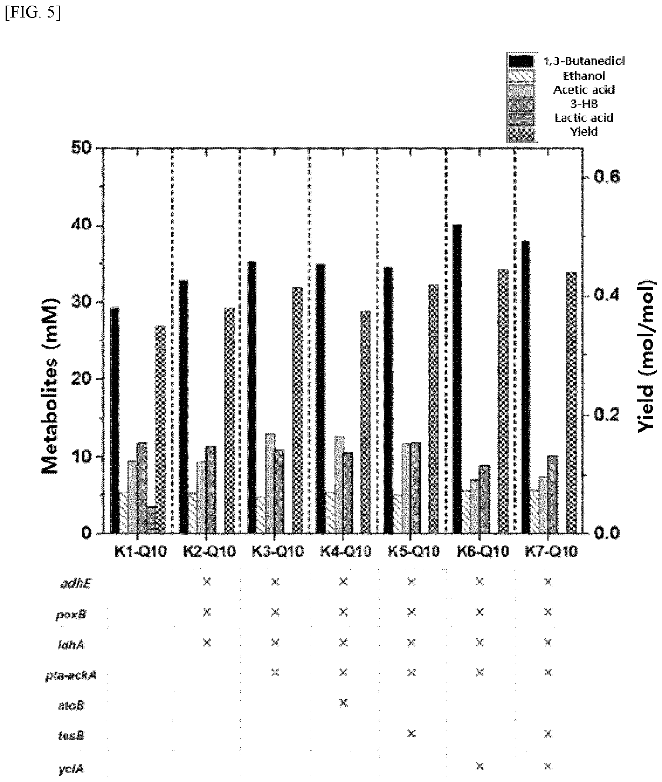

K6Q10 strains (ΔadhE ΔpoxB ΔldhA Δpta-ackA ΔyciA) were selected for further development of strains (FIG. 5).

### <Example 5> TCA cycle and genetic modifications associated with TCA cycles

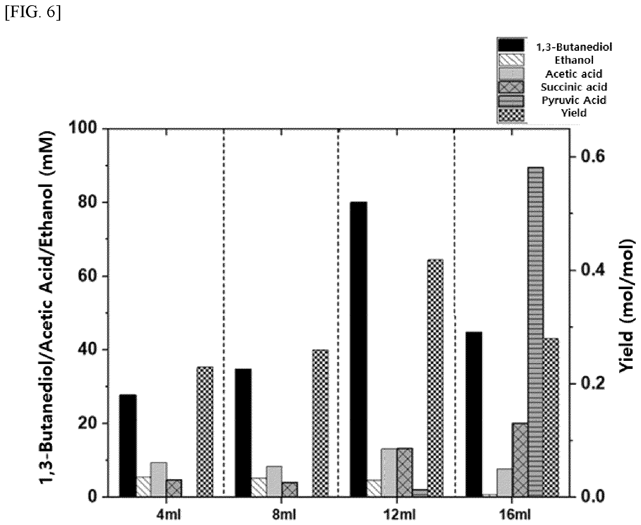

Pyruvic acid is an important metabolic intermediate or precursor through which metabolic pathways such as major glycolysis, TCA cycle, and 1,3-BDO biosynthesis are connected. In general, the accumulation of pyruvic acid indicates that the pathway after pyruvic acid is not functioning properly. When pyruvate begins to accumulate, it in turn blocks the upstream pathway for conversion of glucose to pyruvic acid, and finally both intracellular inflow and catabolism of initial glucose are discontinued. In the fermentation of the K6Q10 strain using a flask, pyruvate accumulated in later half of fermentation when the stirring rate was lowered (FIG. 6). Increased stirring rate lowered accumulation of pyruvate, but at this time, BDO production was reduced due to the high carbon flux into the TCA cycle. The optimal volume of fermentation broth was 12 mL.

Theoretically, if glucose does not enter the TCA cycle through the metabolic process but all of them enter the 1,3-BDO pathway, 1 mole of 1,3-BDO and 1 mole of NADH are produced per mole of glucose. It is necessary to assume that energetically one NADPH is produced from one NADH and one ATP.

1 Glucose + 2 ADP + 3 NAD⁺ + 2 NADPH →1 1,3-BDO + 2 ATP + 3 NADH + 2 NADP⁺ + 2 CO₂

When acetyl-CoA is metabolized through the TCA cycle upon conversion of glucose to 1,3-BDO, a lot of NADH is produced. As a result, redox imbalance occurs. Under anaerobic conditions or oxygen-limited conditions, this redox imbalance may inhibit the activity of LpdA, a subenzyme of pyruvate dehydrogenase (PDH), resulting in pyruvate accumulation and blockage of acetyl-CoA production.

In addition, pyruvate accumulation activates pdhR gene expression, and proteins of this gene suppress the expression of pdh operons, thereby further reducing PDH activity.

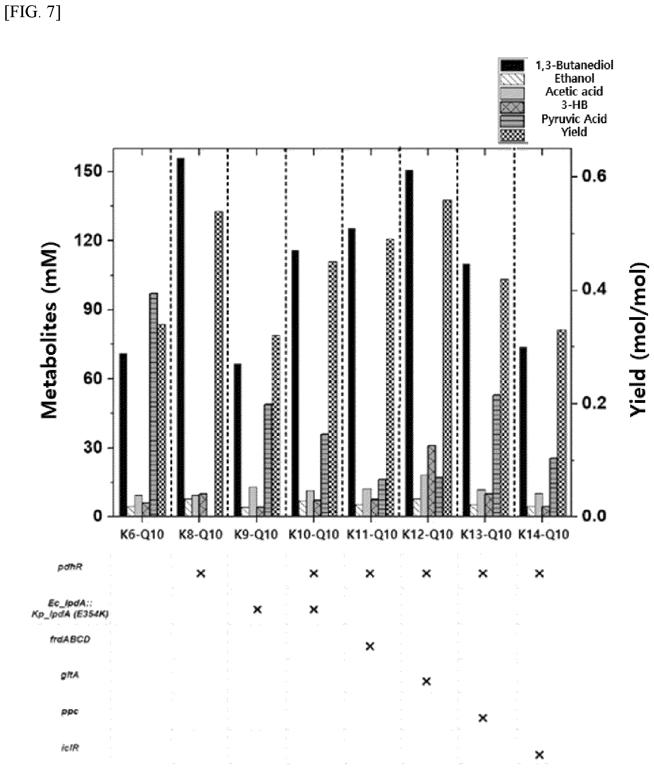

In order to increase PDH activity, K8-Q10 was prepared by removing pdhR, a transcription inhibitor of PDH complex enzymes (FIG. 7). Removal of pdhR reduced pyruvate accumulation and significantly improved 1,3-BDO titer and yield (148 mM & 0.52 mol/mol) at a flask scale. This indicates that PDH activity was a rate determining step in BDO production in previous strains such as K6, and that overexpression of PDH and the high PDH activity thereby are very important for BDO production.

To further enhance PDH activity, used was a mutant lpdA, which encodes mutant LpdA, which is less sensitive to NADH (FIG. 7). BDO production was lower than K8-Q10 when used alone (K9-Q10) or in combination with pdhR deletion (K10-Q10). This suggests that the amount of PDH protein is rate-limiting, rather than inhibition of activity by NADH at the enzymatic level.

The documents showed that when TCA flux was reduced by decreasing the expression of citrate synthase (gltA), the production of both butanol and mevalonate improved. Both butanol and mevalonate use acetyl-CoA as a precursor.

gltA-deleted mutant recombinant E. coli (K12-Q10) exhibited improved production rate and yield for 1,3-BDO in the initial period in the flask (FIG. 7). However, in the late stages of bioconversion, it showed heavy accumulation of pyruvate. Based on pyruvate, the results are interpreted to show the importance of downstream, especially TCA cycle flux.

frdABCD removal was attempted to reduce the flux in the reductive TCA cycle, while the ppc removal was attempted to lower the rate at which PEP is converted to oxaloacetate (OAA) and reduce the flux in the reductive TCA cycle. Flux reduction through the reductive TCA cycle by deletion of frdABCD or ppc (K11-Q11 & K13-Q11) produced pyruvic acid and reduced BDO titers (FIG. 7).

Deletion of iclR (K14-Q10) has been attempted to increase the activity of glyoxylate shunt. The deletion of iclR was also not beneficial for the production of 1,3-BDO. BDO production decreased, and pyruvate accumulated (FIG. 7).

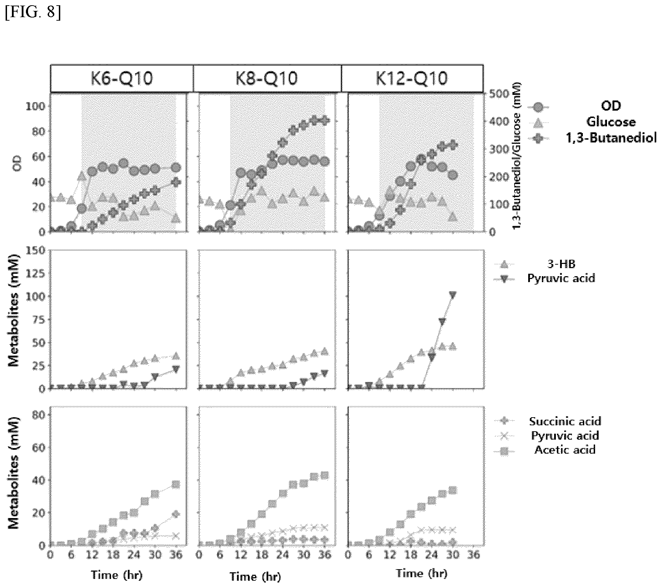

Bioreactor experiments were carried out for pdhR-deleted K8-Q10 strains and gltA-deleted K12-Q10 strains (FIG. 8). The K6-Q10 strain was used as a control. The best results were obtained in the pdhR-deleted K8-Q10 strain. Through 36 hours of fermentation, 1,3-BDO at a concentration of 410 mM (37.4 g/L) was obtained in a yield of 0.48 mol/mol. In comparison, 200 mM (0.31 mol/mol) was obtained in the control K6-Q10, and 312 mM (0.41 mol/mol) was obtained in the K12-Q10 strain from which gltA and pdhR are co-deleted.

### <Example 6> Secondary screening of 3-HBA reductases

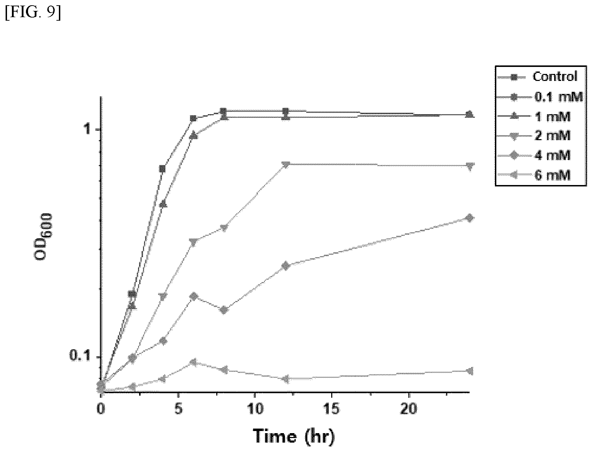

In previous bioreactor experiments, deletion of pdhR(K8) significantly improved 1,3-BDO production to ~410 mM. However, 3-hydroxybutyraldehyde (3-HBA) accumulated in the culture medium at high levels (10 mM). 3-HBA exhibits high toxicity to cell growth at concentrations as low as ~6mM (FIG. 9). Aldehydes are known to inhibit cell growth due to their high reactivity, and toxicity of 3-HBA has been determined to be more toxic than other aldehydes, such as acetaldehyde and propionaldehyde. Accumulation of 3-HBA indicates that the rate of 3-HBA production from 3-HB-CoA is faster than the conversion rate of 3-HBA to 1,3-BDO. In addition, if the conversion of 3-HBA to 1,3-BDO is not rapid, 3-HB may be produced instead of 1,3-BDO. In FIG. 8, all recombinant strains accumulated about 40 mM 3-HB.

Previously, 4 types of enzymes such as Cs_bdh, Ec_yqhD, Kp_dhaT, and Lr_pduQ were tested with 3-HBA reductase (FIG. 3), of which yqhD showing the best performance was used. However, the constant accumulation of 3-HBA and 3-HB indicates a lack of activity in these enzymes. Four aldoketoreductases (AKRs) were tested to improve the conversion from 3-HBA to 1,3-BDO. Two of the selected AKRs were derived from P. denitrificans, one derived from P. putida, and the other from P. aeruginosa. In addition, ygjB and yahK, also known as E. coli-derived alcohol dehydrogenase (ADH), were tested. Thereamong, enzymes coded by ygjB have been reported to have high activity for various aldehydes, especially high activity for butyraldehyde (kcat/Km, 221 sec-1 mM-1), while the enzyme coded by yahK has also been reported to have high activity for various aldehydes, especially high activity for butyraldehyde (kcat/Km, 19.8 sec-1 mM-1). Several ADH genes were cloned into E. coli, mass-produced, isolated and purified, and then tested for the activity of pure enzymes for 3-HBA. Three types of enzymes, Pa_AKR, Ec_YahK, and Ec_YgjB, showed very high activity, with 1.51 U/mg protein, 6.98 U/mg protein, and 238 U/mg protein, respectively (Table 5). In contrast, the Ec_yqhD used so far showed a very low activity of only 0.06 U/mg protein.

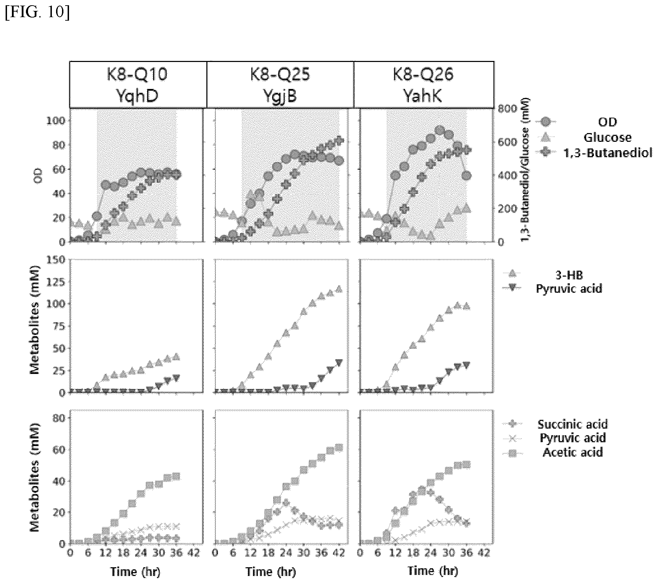

Recombinant E. coli having 3 types of enzymes, YqhD, YgjB, and YahK, were subjected to a bioreactor experiment (FIG. 10). Compared to K8-Q10 strain of YqhD that produced 410 mM (37.4 g/L) 1,3-BDO, the K8-Q25 strain of YgjB yielded 605 mM (56 g/L) through 42 hours of fermentation, and the K8-Q26 strain of YahK yielded a high concentration of 570 mM (51.3 g/L) 1,3-BDO through 42 hours of fermentation (FIG. 10). This shows that the use of good ADH is very important for the production of 1,3-BDO. However, the accumulation of 3-HB did not decrease, but rather increased, and the accumulation of pyruvate occurred in the late fermentation.

**TABLE 5**

| Enzyme | Strain | Purely isolated enzymatic activity (U/mg protein) |
|---|---|---|
| Bdh | *Clostridium acetobutylicum* | N/A |
| YqhD | *E.coli* | 0.06 |
| DhaT | *K. pneumoniae* | 0.44 |
| MCR-N | *Chloroflexus aurantiacus* | ND |
| AKR | *P. denitrificans ATCC 13867* | ND |
| | *P. denitrificans ATCC 19244* | ND |
| | *P. putida* | 0.08 |
| | ***P. aeruginosa*** | **1.51** |
| **YahK** | ***E. coli*** | **6.98** |
| **YgjB (Ahr)** | ***E. coli*** | **238** |

The YgjB enzyme with the best results was selected to investigate the effect of the expression level of this enzyme on the production of 1,3-BDO. To this end, four plasmids including pQ27, pQ28, pQ29, and pQ30 were prepared by weakening the RBS strength (Table 6). The relative strength of RBS was 15% to 189% compared to the control. The newly prepared plasmid was introduced into the K8 host cell, and the flask experiment was performed using the obtained recombinant strain (Table 7). As a result, the pQ-27 plasmid, which had a UTR that was about 50% weaker than that of control pQ25, showed the best results. The final concentration of 1,3-BDO was 160 mM, and the yield was 0.53 mol/mol. YgjB activity in cell extracts was also investigated and was 1.3 U/mg protein in the pQ27 plasmid, which was 43% of the 2.98 U/mg protein as the control pQ25. Future experiments involved the use of pQ27 plasmids.

**TABLE 6**

| | ***K8-Q25 Control*** | ***K8-Q27 (RBS1)*** | ***K8-Q28 (RBS2)*** | ***K8-Q29 (RBS3)*** | ***K8-Q30 (RBS4)*** |
|---|---|---|---|---|---|
| RBS Sequence | **GCAGGAG** AA (SEQ ID NO: 37) | **CATGGAG GT** (SEQ ID NO: 38) | **CGTGGAG GT** (SEQ ID NO: 39) | **CGTGGAG TT** (SEQ ID NO: 40) | **CGTGGAG TT** (SEQ ID NO: 41) |
| Predicted relative UTR intensity (%) | 100 | 66 | 27 | 15 | 189 |

**TABLE 7**

| | ***K8-Q25 Control*** | ***K8-Q27 (RBS1)*** | ***K8-Q28 (RBS2)*** | ***K8-Q29 (RBS3)*** | ***K8-Q30 (RBS4)*** |
|---|---|---|---|---|---|
| Enzymatic activity of cell disrupted fluid (µmol·min⁻¹·mg_protein⁻¹) | 2.98 | 1.30 | 0.49 | 0.43 | 0.33 |
| Concentration of 1,3-BDO | 152.86 | 159.9 | 139.9 | 142.86 | 119.2 |
| Production rate of 1,3-BDO (mmol · hr⁻¹) | 4.21 | 4.43 | 3.79 | 4.21 | 3.44 |
| Yield (mol BDO/mol Glucose) | 0.53 | 0.53 | 0.50 | 0.51 | 0.44 |

### <Example 7> Expansion of NADPH supply

NADPH plays an important role in cell growth and is used as a coenzyme for various enzymes in metabolic processes. Therefore, a sufficient supply of NADPH is crucial to retain cellular and enzymatic activity. In redox reactions, the rate of intracellular enzymatic reactions is often limited by the availability or regeneration rate of cofactors (NADH and/or NADPH) rather than the amount of enzymes.

The stoichiometry for the production of 1,3-BDO from glucose is as follows.

1 Glucose + 2 ADP + 3 NAD+ + 2 NADPH → 1 1,3-BDO + 2 ATP + 3 NADH + 2 NADP+ + 2 CO₂

If NADH is able to be freely converted to NADPH, this conversion reaction may proceed without any problem, but if not, NADPH, which requires 2 moles per 1 mole of 1,3-BDO, becomes a rate determining factor. In other words, if NADPH is scarce, the conversion rate of acetyl-CoA to 1,3-BDO becomes restricted, and, if this slows the TCA cycle down, then pyruvate may accumulate due to NADPH deficiency.

In the glucose metabolism by E. coli, NADPH is produced through the following enzymes or pathways:
1) Transhydrogenase
2) Malic enzyme
3) PP & ED pathways
4) Isocitrate dehydrogenase in the TCA cycle

First, investigation was conducted on the effects of pntAB (Table 8). When pntAB was deleted (K15), 1,3-BDO production was significantly reduced by ~50% compared to the wild-type K8. This suggests that NADPH production by PntAB is very important for BDO production.

Next, for the pntAB, the intrinsic promoter was replaced with a stronger trc promoter (K16) to induce overexpression. RT-PCR showed a 2x enhancement in transcription of pntAB. Unfortunately, however, little has changed in the production of 1,3-BDO. This may be due to the following reasons: (i) PntAB expressed in the wild type has sufficient activity, or (ii) the enzymatic activity of PntAB is not increased after transcriptional upregulation. Experimental results for the production of 1,3-BDO by modification of PntAB demonstrated the importance of NADPH supply, but did not show a quantitative relationship.

**TABLE 8**

| Strain name | Genetic manipulation | Concentration (mM) | Yield (mol/mol) |
|---|---|---|---|
| K8-Q27 | *ΔpoxB ΔldhA Δpta-ackA ΔadhE ΔyciA ΔpdhR* | 152 | 0.54 |
| K15-Q27 | *ΔpntAB* | 75 | 0.48 |
| K16-Q27 | *pntAB*++ | 149 | 0.53 |
| K17-Q27 | *maeB*++ | 144 | 0.51 |
| K17-Q27 | *ΔudhA* | 138 | 0.50 |
| K18-Q27 | *Δpgi* | 30 | 0.19 |
| K19-Q27 | *Δpgi, zwf⁺⁺* | 151 | 0.58 |
| K20-Q27 | *Δpgi, zwf⁺⁺, gnd⁺⁺* | 62 | 0.36 |
| K21-Q27 | *Δpgi, zwf⁺⁺, gnd⁺⁺, Δedd* | 12 | 0.10 |
| K22-Q27 | *Δpgi, zwf⁺⁺, ΔgntR,* | 194 | 0.69 |
| K23-Q27 | *Δpgi, zwf⁺⁺, ΔgntR, edd⁺⁺, eda⁺** | 178 | 0.70 |

In order to further investigate the effects of NADPH, K17 ~ K23 strains were constructed for the effect of multiple pathways/enzymes (Table 8). The second investigation after the pntAB effect was for the upregulation of the maeB gene, which codes for the malic enzyme that produces NADPH while converting malate to pyruvate (K17). The third investigation was to block the Embden-Meyerhoff-Parnas (EMP) pathway and activate the pentose phosphate (PP) or Entner-Doudoroff (ED) pathway in glycolysis. The third strategy was proceeded with various gene deletions/upregulation, such as: (1) The pgi gene encoding phosphoglucose isomerase, an enzyme that converts glucose-6-phosphate to fructose-6-phosphate, was removed in order to block the EMP pathway (K18). (2) The zwf gene encoding glucose-6-phosphate dehydrogenase, which converts glucose-6-phosphate to 6-phophogluconate (6-PG), was upregulated to enhance carbon flux from glucose-6-phosphate nodes to PP pathway and ED pathway (K19). For this purpose, the intrinsic promoter of the ZWF gene was replaced with a stronger trc promoter. (3) In order to enhance the carbon flow from the 6-PG node into the PP pathway, the gnd gene that encodes 6-phophogluconate dehydrogenase, an enzyme that converts 6-PG to ribulose-5-phosphate (Ru5P), was upregulated (K20). For this purpose, the intrinsic promoter of the gnd gene was replaced with a stronger trc promoter. (4) To further enhance the PP pathway, an edd gene encoding phospho-gluconate dehydratase, which is a key enzyme of the ED pathway and converts 6PG to 2-keto-3-deoxy-6-phosphogluconate (KDPG), was additionally removed (K21). (5) In order to facilitate the inflow of carbon from 6PG to the ED pathway, the expression of edd and eda genes was increased by removing gntR which is a protein involved in the ED pathway transcriptional regulation (K22). (6) edd and eda genes were additionally overexpressed to further enhance ED pathway activity (K23). To this end, the intrinsic promoter of the edd/eda gene was replaced with a stronger trc promoter.

pQ27 plasmids were introduced into various variants, and the productivity of 1,3-BDO was investigated. K22-Q27 (194 mM & 0.69 mol/mol) and K23-Q27 (178 mM & 0.71 mol/mol) strains with ED pathway overexpressed showed the best performance. These results show that switching of the carbon flow from the conventional EMP pathway to the ED pathway remarkably increases 1,3-BDO biosynthesis. However, the rate of initial cell growth tended to decrease slightly.

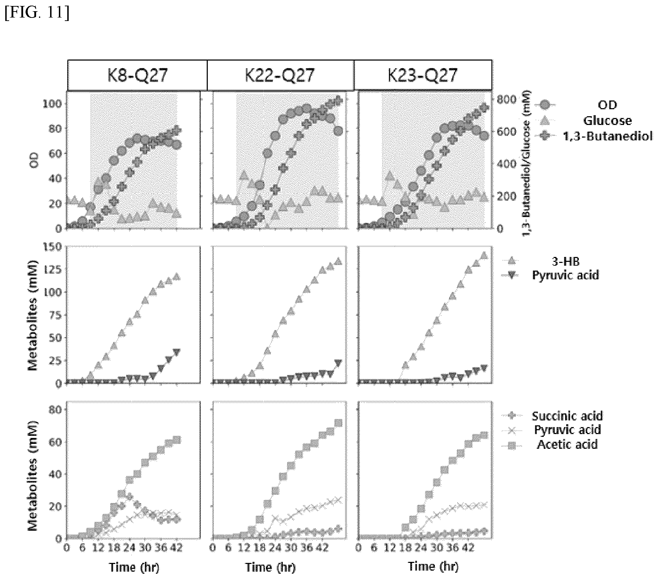

The K22-Q27 and K23-Q27 strains that showed the best performance in the flask experiment were selected, followed by the bioreactor experiment (FIG. 11). The K8-Q27 strain was used as the control. In a bioreactor, the K22-Q27 strain produced 1,3-BDO in a yield of 795 mM (71.2 g/L) and 0.66 mol/mol through fermentation for 48 hours. The K23-Q27 strain also produced 1,3-BDO in a yield of 760 mM (68.2 g/L) and 0.62 mol/mol. In comparison, the control strain, K8-Q27, produced 1,3-BDO in a yield of 620 mM (55.8 g/L) and 0.60 mol/mol (FIG. 11).

### <Example 8> Use of carboxylic acid reductases for conversion of 3-HB

In a bioreactor, K23-Q27 strain produced 1,3-BDO in a yield of 760 mM (68.2 g/L) and 0.62 mol/mol through fermentation for 48 hours. However, this strain produced 140 mM 3-HB as a byproduct. Recently, a result of conversion from 3-HB to 3-HBA using carboxylic acid reductase (CAR) has been reported.

Since the Mm_CAR of Mycobacterium marinum and the Ma_CAR of Mycobacterium abscessus have been reported to be effective in the production of short-chain diol, two types of plasmids, pQ31 (including Mm_CAR) and pQ32 (including Ma_CAR), which convert 3-HB to 1,3-BDO produced by additionally expressing these two enzymes, were developed and introduced into the K23 strain, respectively.

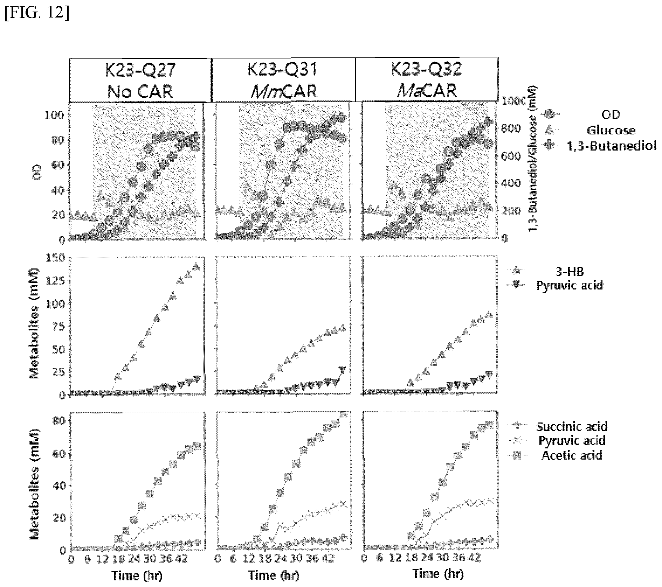

After using the Ma_CAR enzyme in bioreactor experiments, 3-HB production was reduced by 60%, BDO titers were improved to 883 mM, and yields were improved to 0.74 mol/mol (FIG. 12). In addition, after using the Mm_CAR enzyme, 3-HB production was reduced by 40%, BDO titers improved to 825 mM, and yield improved to 0.70 mol/mol. The excellent performance shown by K23-Q31 and K23-Q32 strains with CAR overexpressed led to resolution of issues on the accumulation of 3-HB in the final stage of 1,3-BDO production. 3-HB accumulation intensifies when dissolved oxygen concentrations are high and NADPH is not properly supplied, and K23-Q31 and K23-Q32 strains become commercially advantageous in that they minimize the effects of aeration.

While a specific part of the present disclosure has been described in detail above, it is clear for those skilled in the art that this specific description is merely preferred example embodiments, and the scope of the present disclosure is not limited thereby. Thus, the substantial scope of the present disclosure is defined by the attached claims and their equivalents.

The scope of the present disclosure is presented by the claims described below, and the meaning and scope of the claims, and all modified or altered forms derived from the concept of equivalence should be construed as included in the scope of the present disclosure.

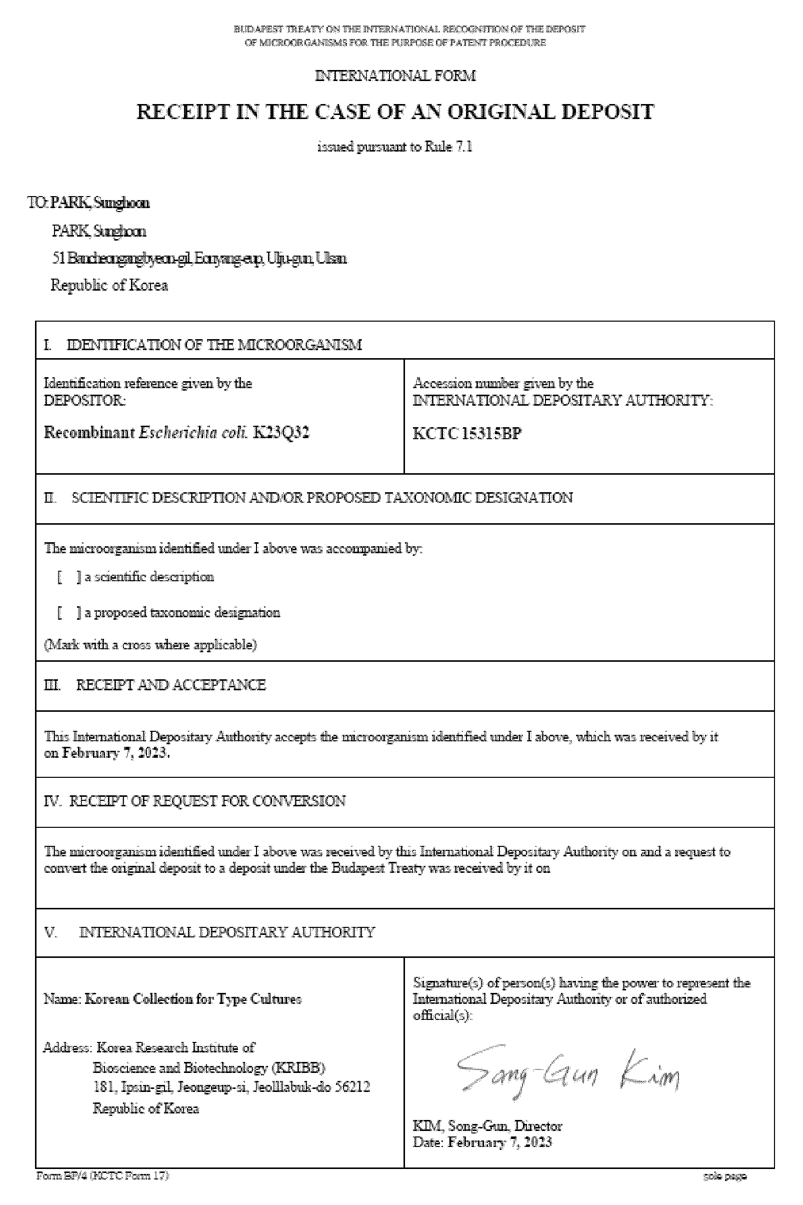

## Claims

1. A recombinant expression vector comprising a butyraldehyde dehydrogenase (bld) gene, an acetyl-CoA acetyltransferase (phaA) gene, an acetoacetyl-CoA reductase (phaB) gene, and a 3-hydroxybutyraldehyde (3-HBA) reductase gene in order.

2. The recombinant expression vector of claim 1, wherein the bld gene comprises a nucleotide sequence represented by SEQ ID NO: 7, the phaA gene comprises a nucleotide sequence represented by SEQ ID NO: 5, and the phaB gene comprises a nucleotide sequence represented by SEQ ID NO: 6.

3. The recombinant expression vector of claim 1, wherein the 3-HBA reductase gene is a yqhD gene comprising a nucleotide sequence represented by SEQ ID NO: 34, a ygjB gene comprising a nucleotide sequence represented by SEQ ID NO: 35, or a yahK gene comprising a nucleotide sequence represented by SEQ ID NO: 36.

4. The recombinant expression vector of claim 3, wherein a ribosome binding site (RBS) in a 5' untranslated region (UTR) of the ygjB gene comprises a nucleotide sequence represented by SEQ ID NO: 37 or SEQ ID NO: 38.

5. The recombinant expression vector of claim 1, wherein the recombinant expression vector further comprises a carboxylic acid reductase (CAR) gene.

6. The recombinant expression vector of claim 5, wherein the CAR gene comprises a nucleotide sequence represented by SEQ ID NO: 20 or SEQ ID NO: 21.

7. A recombinant E. coli strain with enhanced production of 1,3-Butanediol (1,3-BDO), which is transformed with the recombinant expression vector according to any one of claims 1 to 6.

8. The recombinant E. coli strain with enhanced production of 1,3-BDO of claim 7, wherein the recombinant E. coli strain is one obtained by transforming, with the recombinant expression vector, an E. coli strain from which an alcohol dehydrogenase (adhE) gene, a pyruvate oxidase (poxB) gene, a D-lactate dehydrogenase (ldhA) gene, a phosphate acetyltransferase-acetate kinase (pta-ackA) gene, and an acyl-CoA thioesterase (yciA) gene are deleted.

9. The recombinant E. coli strain with enhanced production of 1,3-BDO of claim 8, wherein the recombinant E. coli strain is one obtained by transforming, with the recombinant expression vector, an E. coli strain from which a DNA-binding transcriptional dual regulator (pdhR) gene is additionally deleted.

10. The recombinant E. coli strain with enhanced production of 1,3-BDO of claim 9, wherein the recombinant E. coli strain is one obtained by transforming, with the recombinant expression vector, an E. coli strain from which a citrate synthase (gltA) gene is additionally deleted.

11. The recombinant E. coli strain with enhanced production of 1,3-BDO of claim 9, wherein the recombinant E. coli strain is one obtained by transforming, with the recombinant expression vector, an E. coli strain from which a glucose-6-phosphate isomerase (pgi) gene and a DNA-binding transcriptional repressor (gntR) gene are additionally deleted and in which a NADP+-dependent glucose-6-phosphate dehydrogenase (zwf) gene is additionally overexpressed.

12. The recombinant E. coli strain with enhanced production of 1,3-BDO of claim 11, wherein the recombinant E. coli strain is one obtained by transforming, with the recombinant expression vector, an E. coli strain in which a phosphogluconate dehydratase (edd) gene and a 2-keto-3-deoxygluconate 6-phosphate aldolase (eda) gene are additionally overexpressed.

13. The recombinant E. coli strain with enhanced production of 1,3-BDO of claim 12, wherein the recombinant E. coli strain is a strain deposited under the accession number KCTC 15315BP.

14. A method of enhancing production of 1,3-BDO, comprising culturing the recombinant E. coli strain according to claim 7.
